# EUROPEAN PATENT SPECIFICATION

(11) **EP 2 432 740 B1**
(45) Date of publication and mention of the grant of the patent: **22.02.2017**
(21) Application number: 10719886.3
(22) Date of filing: 14.05.2010
(51) Int. Cl.: C02F 1/76, C02F 1/44, A01N 37/34, C07C 255/23, A01N 37/30, B01D 61/02, B01D 61/04, B01D 61/16, B01D 65/08, B01D 61/14, C02F 1/42

(54) **Controlling bacteria with brominated amide biocidal compounds in a water system at pH higher than 6 as well as new brominated compound**
Bakterienbekämpfung mit bromierter Amid-Verbindung als Biozid in einem Wassersystem bei einem pH-Wert höher als 6, sowie neue bromierte Verbindung
Contrôler des bactéries avec des biocides du type amide bromés dans un système d'eau à un pH supérieur à 6, ainsi qu'un nouveau composé bromé

(30) Priority: 18.05.2009 US 179157 P
(43) Date of publication of application: 28.03.2012
(73) Proprietor: Dow Global Technologies LLC, Midland, MI 48674 (US)
(72) Inventor: GARTNER, Charles, D., Midland MI 48642 (US); YIN, Bei, Buffalo Grove IL 60089 (US); SINGLETON, Freddie, L., St. Charles IL 60174 (US); RAJAN, Janardhanan, S., Glenview IL 60026 (US); GANGULY, Sangeeta, Chicago IL 60611 (US); ROSENBURG, Steven, Shorewood MN 55331 (US); JONS, Steven, D., Eden Prairie MN 55346 (US)
(74) Representative: Beck Greener
(86) International application number: PCT/US2010/034934
(87) International publication number: WO 2010/135192

(56) References cited:
- WO-A1-2008/091453
- US-A- 4 232 041
- US-A1- 2002 128 311

## Description

### Cross-reference to Related Applications

This application claims the benefit of U.S. Provisional Application Ser. No. 61/179,157, filed May 18, 2009.

### Field of the Invention

The invention relates to 2,2-dibromo-2-cyano-N-(3-hydroxypropyl)acetamide and methods of using 2,2-dibromo-2-cyano-N-(3-hydroxypropyl) acetamide or 2,2-dibromomalonamide for the control of bacteria in water systems that have a pH of 6 or greater.

### Background of the Invention

Water systems provide fertile breeding grounds for algae, bacteria, viruses, fungi, and other pathogens. Microbial contamination can create a variety of problems, including aesthetic unpleasantries such as slimy green water, serious health risks such as fungal, bacterial, or viral infections, and clogging or corrosion of equipment.

Biofouling of water systems susceptible to microbial contamination is typically controlled through the use of biocidal agents. For instance, 2,2-dibromo-3-nitrilopropionamide ("DBNPA") is a commercially available biocide that is particularly desirable because it is a fast acting, low cost material that exhibits efficacy against a broad spectrum of microorganisms.

It is known, however, that various physical and/or chemical conditions in the water system can result in the premature deactivation of the biocide, thus rendering the biocide essentially ineffective before the desired microbial control has been achieved. As an example, while DBNPA is stable under acidic conditions, it undergoes rapid hydrolytic degradation in neutral to basic solution. Its rate of disappearance increases by a factor of about 450 in going from pH 6, essentially neutral, to pH 8.9, slightly basic. See, Exner et al., J. Agr. Food. Chem., 1973, 21(5), 838-842 ("Exner"). At pH 8, DBNPA's half life is 2 hours. (Exner, Table 1). At pH 11.3, its half life is only 25 sec, essentially instantaneous degradation. (Exner, page 839, left column). DBNPA, therefore, is not an attractive biocide for use in non-acidic water systems.

It would be a significant advance in the art to provide biocides, for treatment of water systems, that are fast acting, long lasting, and that are stable when subjected to deactivating conditions in the water system, such as increased pH.

US 4,232,041 discloses aqueous antimicrobial compositions which comprise a halogenated amide antimicrobial, such as 2,2-dibromonitrilopropionamide, a water miscible organic solvent such as a straight chain polyalkylene glycol (e.g., polyethylene glycol 200) or an ether thereof (e.g., a mono- or di-lower alkyl and/or phenyl ether) and water and which have improved stability are obtained by ensuring that such compositions are substantially free of glycols having a molecular weight of less than about 70 and of salts of organic acids.

WO 2008/091453 discloses a method of treating water for municipal use comprising treating the feedwater stream on the feed side of a reverse osmosis membrane with a non-oxidizing, bromine-containing biocide in the substantial absence of a reducing agent, such that biofouling of the membrane is reduced or prevented, and measuring the produced water stream on the permeate side for the presence of bromine-containing compounds.

### BRIEF SUMMARY OF THE INVENTION

In one aspect, the invention provides a method for controlling bacteria in a water system having a pH of 6 or greater. The method comprises adding between 1 ppm and 1000 ppm by weight of 2,2-dibromo-2-cyano-N-(3-hydroxypropyl)acetamide or 2,2-dibromomalonamide to the water system.

In another aspect, the invention provides a biocidal compound that is 2,2-dibromo-2-cyano-N-(3-hydroxypropyl)acetamide.

### BRIEF DESCRIPTION OF THE DRAWINGS

FIG. 1 illustrates the biocidal activity of a compound of the invention in an acrylic polymer emulsion.
FIG. 2 compares the biocidal activity of a compound of the invention to commercial compounds in an acrylic polymer emulsion.
FIGS. 3 and 4 illustrate the biocidal activity of a compound of the invention in kaolins.
FIG. 5 illustrates the biocidal activity of a compound of the invention in a calcium carbonate slurry.
FIGS. 6 and 7 compare the biocidal activity of a compound of the invention to commercial compounds in a calcium carbonate slurry.

### DETAILED DESCRIPTION OF THE INVENTION

As noted above, in one aspect the invention relates to methods for controlling bacteria in water systems having pH of 6 or greater. The method comprises adding between 1 ppm and 1000 ppm by weight of 2,2-dibromo-2-cyano-N-(3-hydroxypropyl)acetamide or 2,2-dibromomalonamide to the water system. The inventors have surprisingly discovered that 2,2-dibromo-2-cyano-N-(3-hydroxypropyl)acetamide and 2,2-dibromomalonamide are more resistant to hydrolysis at near-neutral-to-alkaline pH than other biocides, including the commercial compound DBNPA. For instance, the Examples below demonstrate that at pH 6.9 (and a temperature of 30 °C), 2,2-dibromomalonamide (DBMAL), an exemplary compound of the invention, is remarkably more stable than DBNPA (a comparative biocide). No loss of DBMAL is detected over 96 hours whereas 84 % the DBNPA is lost in this same time frame at identical conditions.

The term "2,2-dibromomalonamide" means a compound of the following formula:

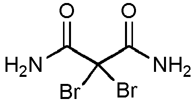

2,2-dibromo-2-cyano-N-(3-hydroxypropyl)acetamide and 2,2-dibromomalonamide can be prepared by those skilled in the art using well known literature techniques.

2,2-dibromo-2-cyano-N-(3-hydroxypropyl)acetamide and 2,2-dibromomalonamide are useful for controlling bacteria in water systems having a pH of 6 or greater. Such water systems include, but are not limited to cooling tower water, metal working fluids, oil and gas field injection or fracturing or produced water or fluids, oil and gas field water-based fluids, paper and pulp mill process water, oil and gas field operation system, oil and gas field transportation system, oil and gas field separation and storage systems, air washers, boiling water, wastewater, ballast water, filtration systems, paint, polymer emulsions, coatings, aqueous-based slurries and dispersed pigments, adhesives, inks, tape joint compounds, household and personal care, or aqueous-based fluids used in leather tanning applications. Preferred water systems are metal working fluids, cooling tower water, paper and pulp mill process water, membrane-based filtration systems, polymer emulsions and aqueous based mineral slurries, such as kaolin, and calcium carbonate slurries.

Representative membrane-based filtration systems include those comprising one or more semi-permeable membranes, including but not limited to: microfiltration, ultrafiltration, nanofiltration, reverse osmosis and ion-exchange membranes. Applicable systems include those comprising a single type of membrane (e.g. microfiltration) and those comprising multiple types of membranes (e.g. ultrafiltration and reverse osmosis). For example, a membrane-based filtration system may comprise an upstream microfiltration or ultrafiltration membrane and a downstream nanofiltration or reverse osmosis membrane.

The subject biocidal compounds may be added to a feed solution prior to filtration, (e.g. added to a storage tank or pond containing feed solution to be treated) or during filtration, (e.g. dosed into a pressurized feed solution during filtration). Moreover, the subject biocidal compounds may be added to cleaning or storage solutions which contact the membrane. For purposes of this description, any aqueous solution (e.g. raw feed water, cleaning solution, membrane storage solution, etc.) contacting a membrane of a system is referred to as a "feed solution."

When used within a system having both micro or ultrafiltration and nanofiltration or reverse osmosis membranes, the subject biocidal compounds provide biocidal effect to each membrane (e.g. both upstream and downstream membranes). Membranes and operating conditions may be chosen to allow the majority of the subject biocidal compounds in a feed solution to permeate (i.e. pass through) the microfiltration and ultrafiltration membranes and be rejected, (i.e. concentrated) by the nanofiltration and reverse osmosis membranes.

In some applications, such as when producing drinking water, it may be advantageous that membranes and operating conditions be selected to pass less than 5%, and more preferably less than 1%, of the subject biocidal compounds into the permeate solution.

The portion of biocidal compound rejected by a membrane(s) may be recovered from the concentrate stream and recycled for use in subsequent treatments, (e.g. directed back to a storage tank or dosed within incoming feed). The recycle of biocidal compounds may be part of an intermittent or continuous process. When the subject biocidal compounds are added to a cleaning solution, membranes within the system may be static soaked in the solution or the solution may flow across the membrane. In the latter case, the solution is preferably recycled to a storage tank. In either case, the intermittent cleaning operation preferably lasts less than 24 hours.

In many membrane-based filtration systems, the pH of the feed solution is at least 7, often at least 8 and in some embodiments the pH of the feed solution is increased to at least 9, and in other embodiments at least 10. Examples of such membrane-based systems are described US 6,537,456 and US 7,442,309. Moreover, membranes of many systems are commonly cleaned or stored with feed solutions having pH values of that have been increased to at least 11 and in some embodiments at least 12. Unlike DBNPA (as described in WO 2008/091453), the subject biocidal compounds remain effective under such neutral and alkaline conditions. As a consequence, the subject biocidal compounds may be added to a wider breath of feed solutions (e.g. pH adjusted aqueous feeds, aqueous cleaning solutions, aqueous storage solutions) used in connection with membrane-based filtration systems.

Modules containing a semipermeable membrane may also be stored for more than a week in the presence of an aqueous solution comprising one of the subject biocidal compounds. The modules and biocidal solution may be contained in bags or tanks. Alternatively, during times when a filtration system is not used, modules may be stored within the filtration system, in contact with the subject biocidal compounds.

The type of membranes used in such systems are not particularly limited and include flat sheet, tubular and hollow fiber. One preferred class of membranes include thin-film composite polyamide membranes commonly used in nanofiltration and reverse osmosis applications, as generally described in US 4,277,344; US 2007/0251883; and US 2008/0185332. Such nanofiltration and/or reverse osmosis membranes are commonly provided as flat sheets within a spiral wound configuration. Non-limiting examples of microfiltration and ultrafiltration membranes include porous membranes made from a variety of materials including polysulfones, polyethersulfones, polyamides, polypropylene and polyvinylidene fluoride. Such micro and ultrafiltration membranes are commonly provided as hollow fibers.

As noted, the pH of water systems in which the compounds of the invention are used have a pH of 6 or greater. In some embodiments, the pH is 7 or greater. In still other embodiments, the pH is 8 or greater.

2,2-dibromo-2-cyano-N-(3-hydroxypropyl)acetamide or 2,2-dibromomalonamide are added to the water system in an amount of between 1 and 1000 ppm, or 5 and 500 ppm, or 5 and 100 ppm by weight is generally adequate. By way of further illustration, for cooling tower water, a typical active dosage of the biocidal compound is 5 ppm to 50 ppm, or 10 ppm to 25 ppm, twice a week or as needed, depending, for instance, on water conditions including biological contamination, chemical water additives, pH, temperature, salinity, and the like. By way of additional illustration, for metal working fluids, a typical active dosage for the biocidal compound is between 20 ppm and 100 ppm, or between 30 ppm and 50 ppm and a frequency of twice a week or as needed, depending on the degree of biological contamination.

2,2-dibromo-2-cyano-N-(3-hydroxypropyl)acetamide or 2,2-dibromomalonamide can be used in the water system with other additives such as, but not limited to, surfactants, ionic/nonionic polymers and scale and corrosion inhibitors, oxygen scavengers, and/or additional biocides.

2,2-dibromo-2-cyano-N-(3-hydroxypropyl)acetamide and 2,2-dibromomalonamide are useful for controlling a wide variety of bacteria. In one embodiment, the bacteria are the *Legionella* species of bacteria, including *Legionella* residing within amoeba. A preferred biocide for this *Legionella* embodiment is 2,2-dibromomalonamide.

*Legionella* have been implicated as the cause of Legionnaires' disease and Pontiac fever, collectively known as legionellosis. Many outbreaks of legionellosis have been attributed to evaporative cooling systems providing infectious doses. *Legionella* exhibit the relatively unique survival ability of parasitizing and residing within amoeba, eventually lysing their host cells to emerge as mature infectious forms. This mechanism has been suggested as the major means of amplification of *Legionella* numbers in natural and man made water systems and their increased virulence. A biocide that can effectively control *Legionella,* including forms of *Legionella* species rendered more virulent by passage through amoeba, is highly desirable. As demonstrated by the examples, 2,2-dibromo-2-cyano-N-(3-hydroxypropyl)acetamide and 2,2-dibromomalonamide, are effective for such bacterial control.

In a second aspect, the invention provides a novel compound that is useful as a biocide for controlling microorganisms. The compound is 2,2-dibromo-2-cyano-N-(3-hydroxypropyl)acetamide.

The compounds described herein are surprisingly resistant to hydrolysis at near-neutral-to-alkaline pH than other biocides, including the commercial compound DBNPA. The compounds consequently are useful for controlling bacteria in a broader range of water systems than currently known biocides and therefore represent a significant advance in the industry.

For the purposes of this specification, the words "control" and "controlling" should be broadly construed to include within their meaning, and without being limited thereto, inhibiting the growth or propagation of bacteria, killing bacteria, disinfection, and/or preservation.

By "hydroxyalkyl" is meant an alkyl group (i.e., a straight and branched chain aliphatic group) that contains 1 to 6 carbon atoms and is substituted with a hydroxyl group. Examples include, but are not limited to, hydroxymethyl, hydroxyethyl, 2-hydroxypropyl, 3-hydroxypropyl, and the like.

"Halogen" refers to fluoro, chloro, bromo, or iodo.

Unless otherwise indicated, ratios, percentages, parts, and the like used herein are by weight.

The following examples are illustrative of the invention but are not intended to limit its scope.

### EXAMPLES

The following compositions are evaluated in the Examples:
2,2-Dibromo-3-nitrilopropionamide ("DBNPA") is obtained from The Dow Chemical Company.
2,2-Dibromomalonamide ("DBMAL") is obtained from Johnson Mathey.
2,2-Dibromo-2-cyano-N-(3-hydroxypropyl)acetamide ("DBCHA") is prepared as shown in Example 1.
CMIT/MIT (5-chloro-2-methyl-4-isothiazolin-3-one and 2-methyl-4-isothiazolin-3-one) is obtained from The Dow Chemical Company.
Glutaraldehyde is obtained from The Dow Chemical Company.
Dioctyl dimethyl ammonium chloride and didecyl dimethyl ammonium chloride are obtained from Lonza Inc.
1-Bromo-3-chloro-5,5-dimethylhydantoin ("BCDMH") is obtained from Lonza Inc..
Triazine (1,3,5-triethylhexahydro-1,3,5-triazine) is obtained from Clariant Corporation.

### Example 1

### Preparation of 2,2-Dibromo-2-cyano-N-(3-hydroxypropyl)acetamide (DBCHA)

0.1 mole of 3-amino-1-propanol (7.51 grams) is added to a solution of 0.1 moles methyl cyanoacetate (10.1 grams) in methanol (40 grams). The mixture is stirred and heated to 60 °C for 30 minutes. The methanol solvent is vacuum stripped from the reaction product. The reaction product, without any further purification necessary, is dissolved in water and reacted with 0.1 mole of bromine (16.0 grams) and 0.03 mole of sodium bromate (5.0 grams), The reaction temperature is kept below 30 °C. After the bromine and sodium bromate addition is complete the reaction mixture is allowed to stir for 30 minutes before neutralizing to pH 3 to 4 with dilute sodium hydroxide. Yield is 0.09 mole of 2,2-dibromo-2-cyano-N-(3-hydroxypropyl)acetamide (28 grams).

### Example 2

### Stability Against Hydrolysis: Comparison of DBMAL and DBNPA

Dilute solutions (less than 0.5 wt %) of DBMAL and DBNPA are prepared at three different pHs. The pH is set and maintained, by using standard buffer solutions, at pH 6.9, 8.0 and 9.0. These solutions are then held at constant temperature at either -1 °C or 30 °C. Periodically, aliquots are analyzed by HPLC to determine the level of DBMAL or DBNPA remaining. Results are shown in Table 1.

**Table 1.**

| | **Three DBNPA Samples** | | | **Three DBMAL Samples** | | |
|---|---|---|---|---|---|---|
| Hours | pH 9, T=-1C | pH 8, T=-1C | pH 6.9, T=30C | pH 9, T=-1C | pH 8, T=-1C | pH 6.9, T=30C |
| 0 | 3842 | 4068 | 3991 | 4576 | 3866 | 3746 |
| 2 | 2818 | 3998 | 4155 | 4022 | 4031 | 4612 |
| 24 | 1256 | 3506 | 2557 | 3891 | 4191 | 3857 |
| 48 | 659 | 3578 | 1361 | 3603 | 4187 | 3935 |
| 72 | 363 | 3149 | 918 | 4018 | 4290 | 3966 |
| 96 | 239 | 3070 | 658 | 3456 | 3883 | 4212 |
| | | | | | | |

| Hours | **Calculated Percent Reduction of the Active Ingredient at Various Times** | | | | | |
|---|---|---|---|---|---|---|
| 48 | 83 | 12 | 66 | 21 | 0 | 0 |
| 72 | 91 | 23 | 77 | 12 | 0 | 0 |
| 96 | 94 | 25 | 84 | 24 | 0 | 0 |

Table 1 shows that even at near-neutral conditions (pH = 6.9) and a temperature of 30 °C, DBMAL is remarkably more stable than DBNPA (a comparative biocide). No loss of DBMAL is detected over 96 hours whereas 84 % the DBNPA is lost in this same time frame at identical conditions.

### Example 3

### Efficacy in Cooling Tower Water: Comparison of DBMAL and DBNPA

DBMAL and DBNPA are added to 50 ml of a cooling tower water sample (at about pH 8.3) in a 125 ml flask containing about 10⁷ CFU/mL of bacteria, at final active concentration of 50 ppm, 25 ppm and 12.5 ppm. The same contaminated cooling tower water sample without biocide is used as control. The mixtures are incubated at 30 °C with shaking (175 RPM) for 96 hrs. At 1 hr, 3 hrs, 24 hrs, 48 hrs, 72 hrs and 96 hrs after the biocide addition, the viable bacteria in the mixtures are enumerated using a serial dilution method. Starting from 24 hrs after the sampling, the mixtures are re-inoculated with about 10⁶CFU/mL of bacteria isolated from the cooling water sample. Table 2 shows the efficacy of DBMAL and DBNPA at different time points, expressed as reductions in numbers of bacteria. Numbers presented are logarithm (base 10) transformations of bacterial counts.

**Table 2. Comparison of the biocidal efficacy of DBMAL and DBNPA against bacteria in Cooling Tower water**

| **Biocide** | | **Bacterial log₁₀ reduction at different time points after biocide addition** | | | | | |
|---|---|---|---|---|---|---|---|
| | | 1hr | 3hr | 24hrs | 48hrs | 72hrs | 96hrs |
| **Active concentratio n** | **Chemical** | | | | | | |
| 50ppm | DBMAL | >=5.3 | 5.5 | >=6. 3 | >=6. 3 | 3.8 | 4.2 |
| | DBNPA* | >=5.3 | >=5. 7 | >=6. 3 | 6.1 | 1.0 | 0.8 |
| 25ppm | DBMAL | 4.3 | >=5. 7 | >=6. 3 | 6.0 | 3.0 | 3.2 |
| | DBNPA* | 5.2 | >=5. 7 | >=6. 3 | 4.2 | 1.0 | 0.7 |
| 12.5ppm | DBMAL | 1.2 | 5.5 | >=6. 3 | 5.2 | 1.2 | 0.8 |
| | DBNPA* | 5.3 | >=5. 7 | >=6. 3 | 1.5 | 0.7 | 0.3 |

| | | | | | | | |
|---|---|---|---|---|---|---|---|
| * comparative example | | | | | | | |

As indicated in Table 2, DBMAL exhibits a relative slower killing action than the comparative compound DBNPA, however, DBMAL's effectiveness (>3 log₁₀ killing) lasts for two days more than that of DBNPA at the same active concentration of 50 ppm and 25 ppm, and lasts for one day more than DBNPA at the same active concentration of 12.5 ppm.

### Example 4

### Efficacy in Cooling Tower Water: Comparison of DBMAL and Other Biocides

Sterile artificial Cooling Tower water (0.2203 g of CaCl₂, 0.1847 g of MgSO₄, and 0.2033 g of NaHCO₃ in 1L water, approximately pH 8.5) is contaminated with field isolated bacteria at about a 10⁷ CFU/mL concentration. The aliquots of this contaminated water are then treated with eight dosage levels of DBMAL and five other commonly used biocides in cooling water application. The same contaminated water sample without biocide is used as control. After incubating at 30 °C for 1 hr, 3 hrs, 24 hrs, 48 hrs, 72 hrs and 96 hrs, the valid bacteria in the aliquots were enumerated using serial dilution method. Starting from 24 hrs after the sampling, all water samples are reinoculated with about 10⁶ CFU/mL of bacteria. Table 3 compares the efficacy of the six biocides at different time points, expressed by bacterial log₁₀ reduction. DBMAL shows high and long lasting efficacy in this comparison study.

**Table 3. Comparison of the biocidal efficacy of six biocides against bacteria isolated from Cooling Tower water**

| Biocides | Minimum biocide dosage (ppm, active) needed for at least 3 log₁₀ bacterial reduction | | | | | |
|---|---|---|---|---|---|---|
| | 1h | 3h | 24h | 48h | 72h | 96h |
| CMIT/MIT* | 7 | 5.0 | 3.0 | 2.0 | 2.0 | 2.0 |
| Glutaraldehyde* | 36.0 | 26.0 | 26.0 | 51.0 | 51.0 | 71.0 |
| Dioctyl dimethyl ammonium chloride* | 29.0 | 29.0 | 56.0 | 56.0 | 79.0 | >79.0 |
| BCDMH (1-bromo-3-chloro-5,5-dimethylhydantoin, active ppm is measured by available bromine & chlorine)* | 7.0 | 26.0 | >70.0 | >70.0 | >70.0 | >70.0 |
| DBNPA* | 8.0 | 8.0 | 11.0 | 15.0 | 15.0 | 21.0 |
| DBMAL | 15.0 | 11.0 | 5.0 | 5.0 | 5.0 | 15.0 |

| | | | | | | |
|---|---|---|---|---|---|---|
| * comparative example | | | | | | |

### Example 5

### Efficacy in a Metal Working Fluid: Comparison of DBMAL and Other Biocides

A semi-synthetic metal working fluid (MWF) (55.35% of deionized water, 20.00% of oil, 15% of sodium sulfonate, 4% of ALKATERGE™ T-IV (surfactant), 3% of oleic acid, 2% of glycol ether, 0.65% of AMP™-95 (neutralizing amine)) is contaminated with field isolated bacteria at about a 10⁶ CFU/mL concentration. The aliquots of this contaminated MWF are treated with four biocides at 8 dosage levels. The same contaminated MWF sample without biocide is used as control. After incubating at room temperature for 2 hr, 4 hrs, 24 hrs, 48 hrs, 72 hrs and 96 hrs, the viable bacteria in the aliquots are enumerated using serial dilution method. Table 4 shows the efficacy of DBMAL and three other biocides against bacteria in MWF. Again, DBMAL shows high and long lasting efficacy in this study. No re-inocculation of field isolates is performed in this example.

**Table 4. Comparison of the biocidal efficacy of four biocides against bacteria isolated from field**

| Biocides | Minimum dosage (ppm, active) required for at least 3 log₁₀ reduction in bacterial counts | | | | | |
|---|---|---|---|---|---|---|
| | 2h | 4h | 24h | 48h | 72h | 96h |
| CMIT/MIT* | 19 | 14 | 14 | 11 | 11 | 8 |
| Triazine* | 1934 | 1487 | 1144 | 1144 | 1144 | 1144 |
| DBNPA* | 42 | 32 | 42 | 55 | 55 | 55 |
| DBMAL | 57 | 44 | 44 | 34 | 26 | 34 |

| | | | | | | |
|---|---|---|---|---|---|---|
| * comparative example | | | | | | |

### Example 6

### Bactericidal Efficacy: DBMAL and DBCHA compared with DBNPA

A sterile synthetic salts water is prepared and inoculated with *Pseudomonas aeruginosa* ATCC 10145 at about 10⁷ CFU/mL. The synthetic water is prepared to contain (in 1 liter deionized water) the following: CaCl₂, 0.2203 g; MgSO₄, 0.1847 g; and NaHCO₃, 0.2033 g. The final pH of the synthetic cooling water is adjusted to 8.5. Aliquots of the cell suspension are then treated with biocides at selected dosage levels. The same contaminated water sample without biocide is used as control. After incubating at 37 °C for different time intervals, numbers of surviving bacteria are enumerated using a serial dilution method. After incubating for 24 hr, all cell suspensions are reinoculated with *Pseudomonas aeruginosa* ATCC 10145 at ∼10⁶ CFU/mL.

The efficacy results for DBMAL (inventive), DBCHA (inventive) and DBNPA (comparative) are shown in Table 5. The data are expressed as the dosage required to achieve a 3 log₁₀ bacterial reduction in water samples after each incubation period.

**Table 5. Bactericidal efficacy of Group 3 DBMAL derivatives against P. aeruginosa ATCC 10145 at different contact time intervals**

| **Biocide s** | **Dosage required for 3 log₁₀ bacterial reduction (ppm, active)** | | | |
|---|---|---|---|---|
| | **1hr** | **3hr** | **24hr** | **48hr** |
| DBMAL | 14.8 | 6.6 | 6.6 | 6.6 |
| DBCHA | 4.4 | 4.4 | 9.9 | 33.3 |
| DBNPA* | 2.9 | 2.9 | 4.4 | 50.0 |

| | | | | |
|---|---|---|---|---|
| * comparative example. | | | | |

The bactericidal efficacy of DBMAL starts slightly later than that of DBNPA, however, its efficacy increases with time and is better than DBNPA after 48 hour incubation and when rechallenged with *P. aeruginosa* cells. DBCHA also shows better long term efficacy than DBNPA.

### Example 7

### Control of Amoeba amplified Legionella

Since *Legionella* are amplified in natural and man made systems, such as cooling towers, by passage through amoeba, eradication of such amoeba fed *Legionella*, is more important and relevant. This example uses amoeba (*Acanthamoeba polypohaga*) fed *Legionella pneumophila* (AfLp) in evaluating suitable biocides.

The *Legionella* are allowed to infect and grow inside amoeba starting with a low multiplicity of infection (1 *Legionella* to 100 amoeba cells). Such a passage is repeated one more time allowing for establishment of the more virulent form as their dominant physiology, prior to exposure to various concentrations of biocides. The evaluations are conducted after two and twenty four hours of exposure. Appropriate neutralization of the biocides is carried out prior to enumeration of survivors. Table 6 below compares effectiveness of various biocides against both AfLp and free normally grown *Legionella* cells.

**Table 6**

| Biocides | Active concentration (ppm) required for complete kill (6 log reduction) | | | |
|---|---|---|---|---|
| | Free *Legionella* | | Amoeba fed *Legionella* | |
| | 2 hours | 24 hours | 2 hours | 24 hours |
| DBNPA | 12.5 | 3.12 | 50 | 25 |
| DBMAL | 6.25 | 1.56 | 12.5 | 3.12 |
| CMIT | 16 | 1 | >64 | 16 |
| Glutaraldehyde | 10 | 5 | 20 | 15 |
| Glutaraldehyde + DDAC | 2.5 | 1.25 | 20 | 10 |
| DDAC | 20 | 15 | 60 | 40 |

| | | | | |
|---|---|---|---|---|
| DDAC = didecyl dimethyl ammonium chloride | | | | |

The data shows that for every biocide tested the amount needed to kill AfLp is greater than the amounts needed to kill free *Legionella.* However the amounts of DBMAL required for *Legionella* control is much lower than those needed for other tested biocides, including DBNPA. This is an unexpected and surprising finding. The levels of DBMAL needed for providing 6 log kills are only about twice that needed for controlling free cells at the corresponding time points. DBMAL provides a means of controlling the more virulent form of AfLp at low dosages when compared to other commonly used biocides.

### Example 8

### Preservation of Polymer Emulsions

Samples of a generic acrylic polymer emulsion (49.9% [w/v] solids, pH 8.2) are inoculated with approximately equal numbers (*ca.* 1 x 10⁶ per ml) of cells of *Pseudomonas aeruginosa, Klebsiella pneumoniae, Bacillus subtilis, and Staphylococcus aureus.* The cell inocula are evenly dispersed in the emulsion samples by vigorous agitation. Samples are then sampled to measure the initial inoculum size. The acrylic polymer emulsions are then amended with selected concentrations of DBMAL (from 2.5 to 500 ppm [or mg/l]). The biocide-treated emulsions are incubated at room temperature (ca. 23 °C) on a rocker platform to provide gentle agitation. Samples for enumerating surviving bacteria are collected after 2, 24, and 48 hr of incubation, serially diluted in phosphate-buffered saline, and plated onto Tryptic Soy Agar (TSA) plates.

### Experiment 1.

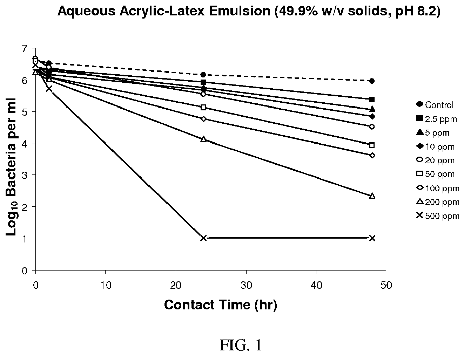

Results. As illustrated in FIG. 1, samples collected after 2-hr contact time showed relatively unchanged bacteria counts in the presence of all but 500 ppm DBMAL. Plate counts obtained after 24-hr contact time showed a clear dose-response relationship. The 48-hr contact time samples illustrated that the biocide continued to inhibit the test organisms during the entire study. The almost linear decrease in numbers of surviving bacteria in samples treated with up to 200 ppm indicated that DBMAL was stable in the emulsion.

### Experiment 2.

In this study, a set of samples of the acrylic polymer emulsion are prepared as described above and treated with DBMAL (0 to 50 ppm) and a second set treated with DBNPA (0 to 50 ppm). After a 48-hr contact time, samples are collected for plate counts.

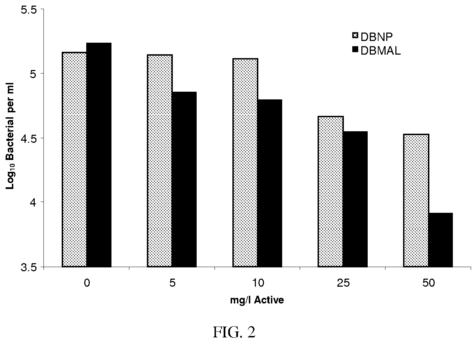

Results. As illustrated in FIG. 2, both DBMAL and DBNPA treatments cause reductions in bacterial counts; the decreases in plate counts are positively correlated to the concentration of biocide added. In all concentrations tested, DBMAL is more efficacious than DBNPA.

### Example 9

### Preservation of Mineral Slurries

### Experiment 1: Efficacy of DBMAL in kaolin.

Two samples of kaolin slurries (Kaolux HS [particle size finer than 2 microns, 83.4%; 64.9% solids; pH 6.6] and Kaogloss [particle size finer than 2 microns, 90.7%; 70.1% solids; pH 6.3]), are obtained from Thiele Kaolin Company, Sandersville, Georgia. Samples of the two slurries are inoculated with four bacterial species (described in Example 8), sampled to confirm the initial size of the bacterial community, and treated with selected concentrations of DBMAL. Samples for bacterial enumeration are collected after 24 hr and 48 hr incubation periods.

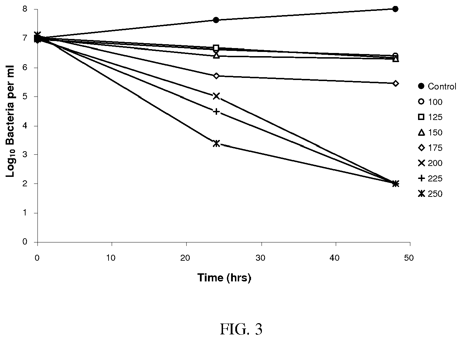

Results. The bacteria inoculated into the Kaolux samples treated with DBMAL are sensitive to the presence of the biocidal active and the response is concentration dependent. For example, as illustrated in FIG. 3, in the absence of DBMAL, there is an increase of approximately one order of magnitude during the 48 hour study. However, in slurries treated with 100 ppm to 150 ppm, the numbers of viable bacteria decrease slightly after 48 hr contact time. A concentration of 175 ppm DBMAL causes a decrease of approximately one order of magnitude. Higher concentrations of DBMAL cause reductions of at least five orders of magnitude.

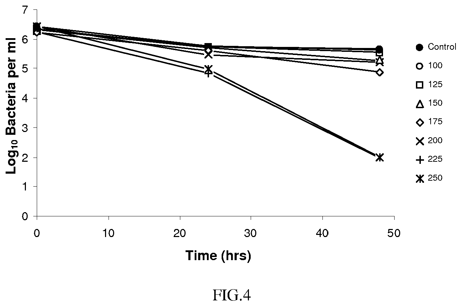

Kaogloss. Samples of Kaogloss are treated as described above with DBMAL and sampled after 24 hr and 48 hr of contact time. The results (see FIG. 4) are similar to those obtained with Kaolux except the impact of the biocide on the size of the bacterial populations after 24 hr contact time is less.

### Preservation of Calcium Carbonate Slurries

### Experiment 1. 10% CaCO₃.

Suspensions of analytical grade (Sigma-Aldrich) calcium carbonate are prepared using deionized water. The pH of the suspensions are adjusted to 7.0, 8.0, and 9.0 with solutions of HCl or NaOH, as appropriate, immediately before the mixed species consortium of bacteria is added (see above for details). Each suspension is vigorously agitated and sampled to determine the initial numbers of bacteria. After sampling for bacterial enumeration, the suspensions are treated with 10 ppm DBMAL, agitated to ensure proper mixing of the biocide and placed on a platform rocker to provide constant agitation. Samples for bacterial enumeration (via plate counts) are collected after 2-hr and 24 hr contact times.

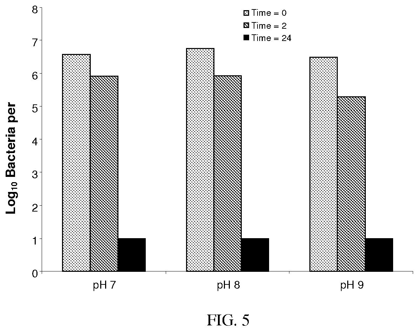

Results. As illustrated in FIG. 5, the 2-hr bacterial counts demonstrate increased inhibition of the bacteria at pH 9.0 versus pH 7.0 and 8.0. After 24 hr contact, all bacterial counts are at the limit of detection (e.g., 1.0 log₁₀ value).

### Experiment 2. Hydrocarb 90 - 76.6% solids, pH 8.7

Aliquots of a sample of the commercially available calcium carbonate slurry, Hydrocarb 90 (Omya), are inoculated with the bacterial consortium as previous described. After agitating to disperse the inoculum, the samples are amended with 5 ppm and 10 ppm DBNPA or DBMAL. The samples are incubated on a rocker platform to provide constant agitation and sampled after 24 hr to determine numbers of bacteria. The initial concentrations of bacteria are higher than in previous examples because of the amount of bacteria present in the sample before the mixed species consortium is added.

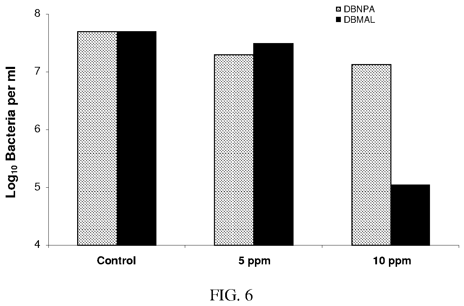

Results. The populations of bacteria in samples treated with 5 ppm of DBNPA or DBMAL decrease relatively little during the 24-hr contact (FIG. 6). However, numbers of bacteria in samples treated with 10 ppm DBMAL decrease by more than 2.5 orders of magnitude while those in the 10 ppm DBNPA-treated slurry decrease by approximately 0.5 order of magnitude. Thus, DBMAL is significantly more efficacious than DBNPA.

### Experiment 3

This study is carried out as previously described except the efficacy of DBMAL is compared to that of DBNPA and CMIT/MIT. For this study, efficacies of the actives are compared using single doses that are typical single dose treatments for products under field conditions.

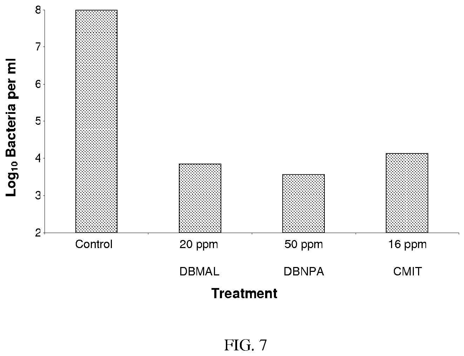

Results. The results (FIG. 7) demonstrate that, following a 24-hr contact time, a single dose of 20 ppm DBMAL is as effective as 50 ppm DBNPA or 16 ppm CMIT. The difference in numbers of bacteria in 20 ppm DBMAL is approximately one-quarter order of magnitude larger than those in the samples that received more than twice as much DBNPA. A similar difference is detected between the DBMAL-treated samples and those that received 16 ppm CMIT.

While the invention has been described above according to its preferred embodiments, it can be modified within the scope of this disclosure. This application is therefore intended to cover any variations, uses, or adaptations of the invention using the general principles disclosed herein. Further, the application is intended to cover such departures from the present disclosure as come within the known or customary practice in the art to which this invention pertains and which fall within the limits of the following claims.

## Claims

1. A method for controlling bacteria in a water system, the method comprising adding between 1 ppm and 1000 ppm by weight of 2,2-dibromo-2-cyano-N-(3-hydroxypropyl)acetamide or 2,2-dibromomalonamide to the water system, wherein the water system has a pH of greater than 6.

2. A method according to Claim 1 wherein the water system has a pH of 7 or greater.

3. A method according to any one of claims 1-2 wherein the water system is: cooling tower water, metal working fluids, oil and gas field injection or fracturing or produced water or fluids, oil and gas field water-based fluids, paper and pulp mill process water, oil and gas field operation system, oil and gas field transportation system, oil and gas field separation and storage systems, air washers, boiling water, wastewater, ballast water, filtration systems, paint, polymer emulsion, coatings, aqueous-based slurries and dispersed pigments, adhesives, inks, tape joint compounds, household and personal care, or aqueous-based fluids used in leather tanning applications.

4. A method according to any one of claims 1-3 wherein the water system comprises a membrane-based filtration system comprising at least one semi-permeable membrane selected from at least one of: microfiltration, ultrafiltration, nanofiltration, reverse osmosis and ion exchange membranes; wherein the method comprises adding the 2,2-dibromo-2-cyano-N-(3-hydroxypropyl)acetamide, 2,2-dibromomalonamide or mixtures thereof to a feed solution followed by contacting the feed solution with the semi-permeable membrane.

5. A method according to claim 4 wherein the membrane-based filtration system comprises at least: i) one microfiltration or ultrafiltration membrane and ii) at least one nanofiltration or reverse osmosis membrane.

6. A method according to any one of claims 4-5 wherein the feed solution has a pH of at least 9.

7. A method according to any one of claims 4-6 wherein the feed solution has a pH of at least 11.

8. A method according to any one of claims 1-7 wherein the bacteria is a species of the genus *Legionella.*

9. A method according to any one of claims 1-8 wherein the bacteria is a species *Legionella* that has reproduced inside living amoeba.

10. A compound that is 2,2-dibromo-2-cyano-N-(3-hydroxypropyl)acetamide.

## Patentansprüche

1. Ein Verfahren zur Bekämpfung von Bakterien in einem Wassersystem, wobei das Verfahren das Zugeben von zwischen 1 Gewichts-ppm und 1000 Gewichts-ppm 2,2-Dibrom-2-cyano-N-(3-hydroxypropyl)acetamid oder 2,2-Dibrommalonamid zu dem Wassersystem beinhaltet, wobei das Wassersystem einen pH-Wert von mehr als 6 aufweist.

2. Verfahren gemäß Anspruch 1, wobei das Wassersystem einen pH-Wert von 7 oder mehr aufweist.

3. Verfahren gemäß einem der Ansprüche 1-2, wobei das Wassersystem Folgendes ist: Kühlturmwasser, Metallverarbeitungsfluide, Öl- und Gasfeldinjektions- oder Fracking- oder Förderwasser oder -fluide, wasserbasierte Öl- und Gasfeldfluide, Papier- und Zellstofffabrikprozesswasser, Öl- und Gasfeldbetriebssystem, Öl- und Gasfeldtransportsystem, Öl- und Gasfeldtrenn- und -speichersysteme, Luftwäscher, Siedewasser, Abwasser, Ballastwasser, Filtrationssysteme, Anstrichmittel, Polymeremulsion, Beschichtungen, wasserbasierte Schlämme und dispergierte Pigmente, Haftmittel, Tinten, Bandfugenverbindungen, Haushalts- und Körperpflegemittel oder wasserbasierte Fluide, die in Ledergerbereianwendungen verwendet werden.

4. Verfahren gemäß einem der Ansprüche 1-3, wobei das Wassersystem ein membranbasiertes Filtrationssystem beinhaltet, das mindestens eine semipermeable Membran beinhaltet, ausgewählt aus mindestens einem von Folgendem: Mikrofiltration, Ultrafiltration, Nanofiltration, Umkehrosmose- und Ionenaustauschmembranen; wobei das Verfahren das Zugeben des 2,2-Dibrom-2-cyano-N-(3-hydroxypropyl)acetamids, 2,2-Dibrommalonamids oder Mischungen davon zu einer Zufuhrlösung beinhaltet, gefolgt von dem In-Kontakt-Bringen der Zufuhrlösung mit der semipermeablen Membran.

5. Verfahren gemäß Anspruch 4, wobei das membranbasierte Filtrationssystem mindestens Folgendes beinhaltet: i) eine Mikrofiltrations- oder Ultrafiltrationsmembran und ii) mindestens eine Nanofiltrations- oder Umkehrosmosemembran.

6. Verfahren gemäß einem der Ansprüche 4-5, wobei die Zufuhrlösung einen pH-Wert von mindestens 9 aufweist.

7. Verfahren gemäß einem der Ansprüche 4-6, wobei die Zufuhrlösung einen pH-Wert von mindestens 11 aufweist.

8. Verfahren gemäß einem der Ansprüche 1-7, wobei die Bakterien eine Spezies der Gattung *Legionella* sind.

9. Verfahren gemäß einem der Ansprüche 1-8, wobei die Bakterien eine *Legionella-*Spezies sind, die sich innerhalb einer lebenden Amöbe reproduziert haben.

10. Eine Verbindung, die 2,2-Dibrom-2-cyano-N-(3-hydroxypropyl)acetamid ist.

## Revendications

1. Une méthode pour lutter contre des bactéries dans un système d'eau, la méthode comprenant l'ajout d'entre 1 ppm et 1 000 ppm en poids de 2,2-dibromo-2-cyano-N-(3-hydroxypropyl)acétamide ou de 2,2-dibromomalonamide au système d'eau, dans laquelle le système d'eau a un pH supérieur à 6.

2. Une méthode selon la revendication 1 dans laquelle le système d'eau a un pH de 7 ou plus.

3. Une méthode selon l'une quelconque des revendications 1 à 2 dans laquelle le système d'eau est : de l'eau de tour de refroidissement, des fluides d'usinage de métaux, des fluides ou de l'eau de production ou de fracturation ou d'injection pour champ de pétrole et de gaz, des fluides à base d'eau pour champ de pétrole et de gaz, de l'eau de traitement d'usine de papier et pâte à papier, un système d'opération pour champ de pétrole et de gaz, un système de transport pour champ de pétrole et de gaz, des systèmes de stockage et de séparation pour champ de pétrole et de gaz, des laveurs d'air, de l'eau bouillante, des eaux usées, de l'eau de ballast, des systèmes de filtration, une peinture, une émulsion de polymère, des enduits, des suspensions épaisses à base d'eau et des pigments dispersés, des adhésifs, des encres, des pâtes pour joints à bande, des produits de ménage et de soins d'hygiène personnelle, ou des fluides à base d'eau utilisés dans des applications de tannage de cuir.

4. Une méthode selon l'une quelconque des revendications 1 à 3 dans laquelle le système d'eau comprend un système de filtration membranaire comprenant au moins une membrane semi-perméable sélectionnée parmi au moins un élément d'entre : des membranes de microfiltration, d'ultrafiltration, de nanofiltration, d'osmose inverse et échangeuses d'ions ; la méthode comprenant l'ajout de 2,2-dibromo-2-cyano-N-(3-hydroxypropyl)acétamide, de 2,2-dibromomalonamide ou des mélanges de ceux-ci à une solution d'alimentation suivi par la mise en contact de la solution d'alimentation avec la membrane semi-perméable.

5. Une méthode selon la revendication 4 dans laquelle le système de filtration membranaire comprend au moins : i) une membrane de microfiltration ou d'ultrafiltration et ii) au moins une membrane de nanofiltration ou d'osmose inverse.

6. Une méthode selon l'une quelconque des revendications 4 à 5 dans laquelle la solution d'alimentation a un pH d'au moins 9.

7. Une méthode selon l'une quelconque des revendications 4 à 6 dans laquelle la solution d'alimentation a un pH d'au moins 11.

8. Une méthode selon l'une quelconque des revendications 1 à 7 dans laquelle la bactérie est une espèce du genre *Legionella.*

9. Une méthode selon l'une quelconque des revendications 1 à 8 dans laquelle la bactérie est une espèce *Legionella* qui s'est reproduit au-sein d'une amibe vivante.

10. Un composé qui est le 2,2-dibromo-2-cyano-N-(3-hydroxypropyl)acétamide.
